(19) Europäisches Patentamt
European Patent Office
Office européen des brevets

(11) Publication number: **0 091 181**
**B1**

(12) # EUROPEAN PATENT SPECIFICATION

(45) Date of publication of patent specification: **17.12.86**

(21) Application number: **83300536.6**

(22) Date of filing: **03.02.83**

(51) Int. Cl.[4]: **C 07 D 487/04,** A 61 K 31/40 // (C07D487/04, 209:00, 209:00)

(54) **Aroyl pyrrolizine compounds, process for their preparation and pharmaceutical compositions containing them.**

(30) Priority: **25.02.82 GB 8205546**

(43) Date of publication of application: **12.10.83 Bulletin 83/41**

(45) Publication of the grant of the patent: **17.12.86 Bulletin 86/51**

(84) Designated Contracting States: **BE CH DE FR GB IT LI NL SE**

(58) References cited:
**EP-A-0 053 021**
**US-A-4 097 579**
**US-A-4 232 038**

(73) Proprietor: **BEECHAM GROUP PLC**
**Beecham House Great West Road**
**Brentford Middlesex TW8 9BD (GB)**

(72) Inventor: **Goudie, Alexander Crossan**
**1 Hildersham Close St. Peters**
**Broadstairs Kent (GB)**
Inventor: **Ward, Robert William**
**332 Willowfield Tower**
**Harlow Essex (GB)**

(74) Representative: **Stott, Michael John et al**
**European Patent Attorney**
**Beecham Pharmaceuticals**
**Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 091 181 B1

**Description**

This invention relates to novel compounds having pharmacological activity, to processes of their preparation, to pharmaceutical compositions containing them, and to their use in the treatment of mammals.

Compounds of formula (A),

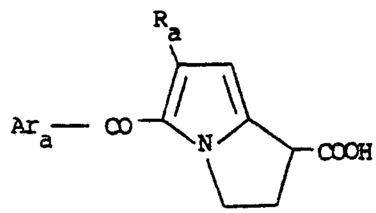

(A)

and their pharmaceutically acceptable esters and salts, wherein $Ar_a$ is phenyl optionally substituted in the *o-*, *m-* or *p*-position by $C_{1-4}$ alkyl, $C_{1-4}$ alkoxy, bromo, chloro or fluoro, 2-pyrryl optionally N-substituted by $C_{1-4}$ alkyl, 2-furyl or 2-thienyl either being optionally substituted in the 3-, 4- or 5-position by methyl, chloro or bromo, or 3-furyl or 3-thienyl, and Ra is hydrogen or $C_{1-4}$ alkyl, are described in US patent Nos. 4 087 539, 4 089 969 and 4 097 574, European patent publication 649, and Belgian patent 865247. Such compounds are described as having various pharmacological activities, including anti-inflammatory and analgesic activity.

A class of compounds have now been discovered which compounds are aroylpyrrolopyrroles with a non-acidic side chain. Such compounds moreover have been found to posses anti-inflammatory and analgesic activity and, because the side-chain is non-acidic, a low gastric irritancy.

Accordingly the present invention provides a compound of the formula (I)

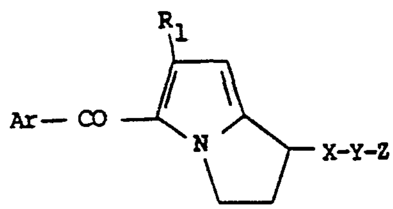

(I)

wherein:

Ar is phenyl optionally substituted in the *o-*, *m-* or *p*-position by $C_{1-4}$ alkyl, $C_{1-4}$ alkoxy, halo, $C_{1-4}$ alkylthio or trifluoromethyl, 2-pyrryl optionally N-substituted by $C_{1-4}$ alkyl, 2-furyl or 2-thienyl either being optionally substituted in the 3-, 4-, or 5- position by $C_{1-4}$ alkyl, chloro or bromo, 3-furyl, or 3-thienyl;

$R_1$ is hydrogen, $C_{1-4}$ alkyl, fluoro, bromo or chloro; and

X is $CH_2$ and either Y is CO, CHOH, $CH(OCOR_2)$ or $C(OR_3)OR_4$, and Z is $CH_3$, or Y and Z together are $C(OR_5)=CH_2$ or $C(OCOR_5)=CH_2$, or X is CHOH, Y is CHOH or CO and Z is $CH_3$, or X and Y together are $CH=C(OR_5)$ or $CH=C(OCOR_5)$ and Z is $CH_3$, in which $R_2$ is phenyl optionally substituted in the *o-*, *m-* or *p*-position by $C_{1-4}$ alkyl, $C_{1-4}$ alkoxy or halo, or is $C_{1-7}$ alkyl optionally substituted by phenyl or amino, $R_3$ and $R_4$ are $C_{1-4}$ alkyl or together are $C_{2-3}$ polymethylene, and $R_5$ is $C_{1-4}$ alkyl.

When Ar is phenyl, examples of optional substituents include chloro, bromo, methyl, methoxy, trifluoromethyl and methylthio. Normally, when phenyl is substituted, it is monosubstituted, particularly by methyl or chloro. Preferably, however, phenyl is unsubstituted.

When Ar is 2-pyrryl, an example of an N-substituent is methyl. Preferably, 2-pyrryl is N-substituted by methyl.

When Ar is 2-thienyl or 2-furyl, examples of optional substituents include methyl, ethyl, chloro and bromo. Preferably, 2-thienyl or 2-furyl are unsubstituted.

Of the various aromatic groups for Ar, phenyl optionally substituted as hereinbefore defined is preferred, particularly unsubstituted phenyl.

The side-chains comprised by the definition for X—Y—Z are of sub-formulae (i) to (x):

| | |
|---|---|
| $CH_2COCH_3$ (i) | $CH_2—C(OCOR_5)=CH_2$ (vi) |
| $CH_2CHOHCH_3$ (ii) | $CHOHCHOHCH_3$ (vii) |
| $CH_2CH(OCOR_2)CH_3$ (iii) | $CHOHCOCH_3$ (viii) |
| $CH_2C(OR_3)(OR_4)CH_3$ (iv) | $CH=C(OR_5)CH_3$ (ix), and |
| $CH_2C(OR_5)=CH_2$ (v) | $CH=C(OCOR_5)CH_2$ (x) |

wherein $R_2$ to $R_5$ are as hereinbefore defined.

Examples of $R_2$ include methyl, ethyl, aminomethyl (as a hydrochloride salt, for example) and phenyl. Preferably $CH_2$ is methyl or aminomethyl.

Preferred examples of $R_3$, $R_4$ and $R_5$ are methyl and ethyl, particularly methyl.

Particular examples of side chains (X—Y—Z) are those wherein X is $CH_2$, Y is CO and Z is $CH_3$ (formula (i)), X is $CH_2$, Y is CHOH and Z is $CH_2$ (formula (ii)), X is $CH_2$, Y is $CH(OCOR_2)$ and Z is $CH_3$ (formula (iii)), X is $CH_2$, Y is $C(OR_3)(OR_4)$ and Z is $CH_3$ (formula (iv)), X is CHOH, Y is CHOH and Z is $CH_3$ (formula (vii)), and wherein X is CHOH, Y is CO and Z is $CH_3$ (formula (viii)). Within the sub-class, $R_2$ is preferably methyl, and $R_3$ and $R_4$ together are preferably ethylene.

Preferably $R_1$ is hydrogen.

The compounds of the present invention exist as isomers. All such isomers, whether individually or as mixtures, are included within the scope of the invention.

The present invention also provides a process of preparing a compound of formula (I), which comprises oxidising a compound of formula (II),

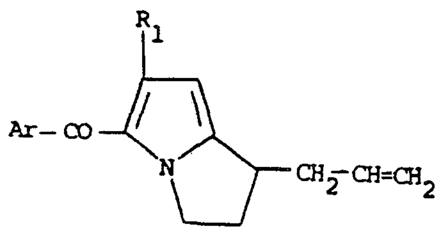

(II)

wherein Ar and $R_1$ are as hereinbefore defined, to give a compound of formula (I), wherein X—Y—Z is of formula (i); optionally reacting the resulting compound with:

(a) a reducing agent to give a compound of formula (I), wherein X—Y—Z is of formula (ii), and optionally reacting a compound of formula (I); wherein X—Y—Z is of formula (ii), with an acylating agent containing the acyl group, $R_2CO$, $R_2$ being as defined hereinbefore, to give a compound of formula (I), wherein X—Y—Z is of formula (iii);

(b) a ketalising agent containing the alkoxy groups, $OR_3$ and $OR_4$, $R_3$ and $R_4$ being as defined hereinbefore, to give a compound of formula (I), wherein X—Y—Z is of formula (iv);

(c) an enol $C_{1-4}$ etherifying agent to give compounds of formula (I), wherein X—Y—Z is of formula (v) and (ix), and separating the compound of formula (I), wherein X—Y—Z is of formula (v), from the compound of formula (I), wherein X—Y—Z is of formula (ix); optionally reacting a compound of formula (I), wherein X—Y—Z is of formula (ix), with an oxidising agent to give a compound of formula (I), wherein X—Y—Z is of formula (viii), and optionally reacting a compound of formula (I), wherein X—Y—Z is of formula (viii), with a reducing agent to give a compound of formula (I), wherein X—Y—Z is of formula (vii);

(d) an enol tri-$C_{1-4}$ alkylsilyl etherifying agent to give a compound of formula (I), wherein X—Y—Z is of formula $CH{=}C(OR_x)CH_3$, $R_x$ being tri-$C_{1-4}$ alkylsilyl, and reacting the resulting ether with an oxidising agent to give a compound of formula (I), wherein X—Y—Z is of formula (viii); or

(e) an enol $C_{2-5}$ esterifying agent to give compounds of formula (I), wherein X—Y—Z is of formula (vi) and (x), and separating the compound of formula (I), wherein X—Y—Z is of formula (vi), from the compound of formula (I), wherein X—Y—Z is of formula (x).

The oxidation of a compound of formula (II) may be carried out in, for example, aqueous dimethylformamide in the presence of palladium chloride and cuprous chloride using pure oxygen or air. Generally, it is sufficient to pass air through the reaction mixture at an ambient or slightly elevated temperature to bring about complete oxidation of the starting material. The resulting compound may be isolated from the reaction mixture by diluting the mixture with water and then extracting the desired compound into a water-immiscible solvent, such as chloroform, which may then be dried and evaporated. The crude product may be purified, if desired, chromatographically using a column of silica gel.

The reduction of the compound of formula (I), wherein X—Y—Z is of formula (i) to a compound of formula (I), wherein X—Y—Z is of formula (ii), may be carried out with a complex hydride reducing agent, such as sodium borohydride, using mild conditions, such as a low temperature and avoiding an excess of reducing agent so as not to reduce the carbonyl group of the Ar—CO moiety.

The acrylating agent employed in the optional reaction with a compound of formula (I), wherein X—Y—Z is of formula (ii), to give a compound of formula (I), wherein X—Y—Z is of formula (iii), is usually a carboxylic acid or derivative thereof, such as an acid chloride, acid anhydride or an active acid ester. The acylation reaction is carried out under conventional conditions, such as described in UK patent No. 1 538 473, in a conventional organic solvent, such as tetrahydrofuran, dioxane, methylene chloride, chloroform, pyridine or toluene. If a non-basic solvent is employed, then an acid acceptor, such as pyridine or triethylamine, should be present during the reaction. The reaction temperature is usually ambient temperature.

The ketalising agent used in the reaction with a compound of formula (I), wherein X—Y—Z is of formula (i), to give a compound of formula (I), wherein X—Y—Z is of formula (iv), is normally a $C_{1-4}$ alkyl

orthoester, such as orthoformate, or a $C_{1-4}$ alcohol or $C_{2-3}$ diol. When an orthoester is employed, the reaction conditions should be non-acidic since otherwise the enol ether will form. Generally, the reaction is carried out under conventional ketalisation conditions, such as described in UK patent No. 1 497 109, using an inert solvent, such as benzene or toluene, at an elevated temperature. It is usually advantageous in ketalation reactions to employ a dehydration catalyst, such as p-toluenesulphonic acid.

Enol $C_{1-4}$ etherification of a compound of formula (I), wherein X—Y—Z is of formula (i), to give compounds of formulae (I), wherein X—Y—Z is of formula (v) and (ix), may be carried out using a conventional etherification agent and conditions, such as those described in US patent Nos. 4 180 585 and 4 200 645. Conventional etherification agents include $C_{1-4}$ alkyl orthoester, such as $C_{1-4}$ alkyl orthoformate, and isopropenyl $C_{1-4}$ alkanoate. The $C_{1-4}$ alkyl orthoester is usually reacted with the compound of formula (I), wherein X—Y—Z is of formula (i), in an alcohol, corresponding to the $C_{1-4}$ alkyl orthoester, for example ethanol and an ethyl orthoester, in the presence of an acid, preferably saturated ethereal hydrogen chloride. The isopropenyl $C_{1-4}$ alkanoate is usually reacted with the compound of formula (I), wherein X—Y—Z is of formula (i), in the presence of p-toluenesulphinic acid at reflux temperature.

The resulting mixture of compounds of formula (I), wherein X—Y—Z is of formulae (v) and (ix), are separated conventionally using, for example, fractional distillation or column chromatography.

The oxidation of a compound of formula (I), wherein X—Y—Z is of formula (ix), to give a compound of formula (I), wherein X—Y—Z is of formula (viii), is carried out using a conventional oxidising agent and conditions, such as metachloroperbenzoic acid and osmium tetroxide/morpholine oxide, in a solvent, such as dichloromethane, at room temperature.

The reduction of a compound of formula (I), wherein X—Y—Z is of formula (viii), to give a compound of formula (I), wherein X—Y—Z is of formula (vii) may be carried out using, for example, sodium borohyride in the manner as described hereinbefore.

the enol tri-$C_{1-4}$ alkylsilyl etherification of a compound of formula (I), wherein X—Y—Z is of formula (i), to give the tri-$C_{1-4}$ alkylsilyl ether of the compound of formula (I), wherein X—Y—Z is of formula (ix), may be carried out in a solvent, such as dichloromethane, using, for example, tri-$C_{1-4}$ alkylsilyl iodide, in particular, trimethylsilyl iodide. The subsequent oxidation may be carried out using the same oxidising agent and conditions as described hereinbefore in relation to the oxidation of a compound of formula (I), wherein X—Y—Z is of formula (ix).

The enol $C_{2-5}$ esterification of a compound of formula (I), wherein X—Y—Z is of formula (i), to give compound of formula (I), wherein X—Y—Z is of formula (vi) and (x), may be carried out using a conventional esterification agent and conditions, for example, as described in US patent No. 4 180 585.

The resulting mixture of compound of formula (I), wherein X—Y—Z is of formula (vi) and (x) are separated conventionally using, for example, fractional distillation or column chromatography.

A compound of formula (II) may be prepared by decarboxylating a compound of formula (III),

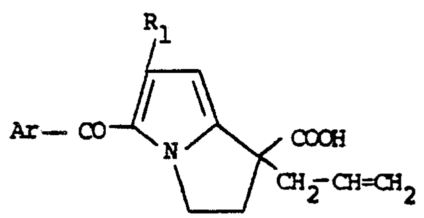

(III)

wherein Ar and $R_1$ are as hereinbefore defined.

Decarboxylation may be carried out simply by heating the compound of formula (II) or by heating it with copper and quinoline at an elevated temperature, for example, 170—210°C.

A compound of formula (III) may be prepared by allylating a compound of formula (IV),

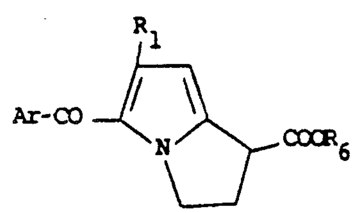

(IV)

wherein Ar and $R_1$ are as hereinbefore defined, and $R_6$ is $C_{1-4}$ alkyl or hydrogen, and, in the case when $R_6$ is $C_{1-4}$ alkyl, hydrolysing the resulting ester to give the free carboxylic acid.

Allylation may be carried out by generating the anion of a compound of formula (IV) by using sodium hydride in dimethoxyethane or, preferably, lithium di-isopropylamine (LDA) in tetrahydrofuran and then quenching the anion with allyl bromide.

Hydrolysis of the ester of formula (IV) may be carried out with normal sodium hydroxide solution followed by neutralisation with hydrochloric acid.

Compounds of formula (IV) are known or can be prepared analogously to the preparation of known compounds, for example, as described in US patent Nos. 4 087 538, 4 089 969 and 4 097 574, European patent Publication 649 and Belgian patent 865247.

The present invention provides a second although less preferred process of preparing a compound of formula (I), wherein X—Y—Z is of formula (v), which comprises decarboxylating a compound of formula (V),

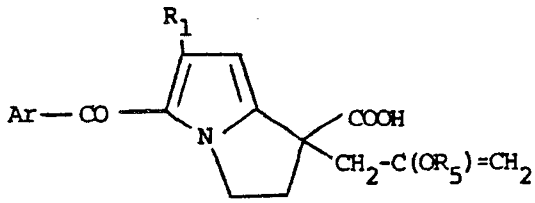

(V)

wherein Ar, $R_1$ and $R_5$ are as hereinbefore defined.

Decarboxylation may be carried out with or without a solvent, such as quinoline, at a temperature of 160 to 200°C, preferably in the presence of copper bronze.

A compound of formula (V) may be prepared by $C_{1-4}$ alkoxyallylation of a compound of formula (IV), as hereinbefore defined.

The intermediates of formulae (II), (III) and (V) are novel and represent part of the present invention. Collectively they are of formula (VI),

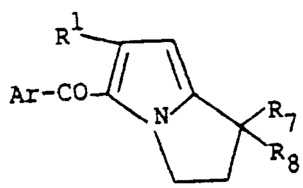

(VI)

wherein Ar and $R_1$ are as hereinbefore defined, and $R_7$ is carboxy and $R_8$ is $CH_2$—$CH{=}CH_2$ or $CH_2$—$C(OR_5){=}CH_2$, $R_5$ being as hereinbefore defined, or $R_7$ is hydrogen and $R_8$ is CH—$CH{=}CH_2$.

The present invention further provides a pharmaceutical composition, which comprises a compound of formula (I) and a pharamceutically acceptable carrier.

A composition of this invention is useful in the treatment of rheumatism and arthritis and in the treatment of pain and other inflammatory conditions.

A composition of the invention, which may be prepared by admixture, may contain a diluent, binder, filler, disintegrant, flavouring agent, colouring agent, lubricant, preservative or the like in conventional manner. These conventional excipients may be employed in conventional manner, for example as in the preparation of compositions of ketoprofen, indomethacin, naproxen, acetylsalicyclic acid and other analgesic or anti-inflammatory agents.

A composition of the invention may be adapted for oral, rectal or parenteral — intravenous or intramuscular — administration but oral administration is preferred.

A composition of the invention will preferably be in the form of a unit dose, such as a tablet or capsule or a sachet containing reconstitutable powder. A unit dose will generally contain from 20 to 1000 mg and preferably will contain from 30 to 500 mg, in particular 50, 100, 150, 200, 250, 300, 350, 400, 450 or 500 mg. The composition may be administered once or more times a day, for example 2, 3 or 4 times daily, so that the total daily dose for a 70 kg adult will normally be in the range 100 to 3000 mg. Alternatively the unit dose will contain from 2 to 20 mg of a compound of the invention and be administed in multiples, if desired, to give the preceeding daily dose.

A particular composition of the invention is a hard gelatin capsule containing the required amount of a compound of the invention in the form of a powder or granulate in intimate mixture with a lubricant, such as magnesium stearate, a filler, such as microcrystalline cellulose, and a disintegrant, such as sodium starch glycollate.

The present invention additionally provides a compound, pharmaceutically acceptable salt or composition of the invention for use in the treatment of inflammatory and/or painful conditions.

The following Descriptions and Examples illustrate the prĕparation of compounds of the invention and the following biological data illustrates their pharmacological, in particular anti-inflammatory, activity.

Description 1

[2,3-Dihydro-1-(2-propenyl)-1H-pyrrolizine-5-yl]-phenylmethanone

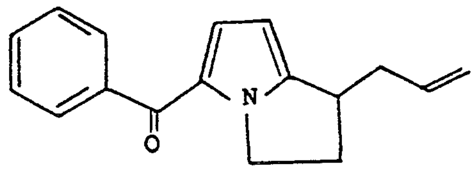

(D1)

5-Benzoyl-2, 3-dihydro-1H-pyrrolizine-1-carboxylic acid (400 mg, 0.0016 mole) was taken up in dry tetrahydrofuran (5 ml) and added dropwise at −20° to a solution of di-isopropylamine (0.53 ml, 0.0038 mole) and butyllithium (2.2 ml of 1.6 M solution in hexane, (0.0035 mole) in dry tetrahydrofuran (10 ml) under nitrogen.

The resulting solution was allowed to come to room temperature over 1½ hours before allyl bromide (0.28 ml, 0.0032 mole) was added. The reaction mixture was then left stirring at room temperature overnight.

On cooling in an ice bath, water (5 ml) was added to the solution, which was then acidified with dilute aqueous hydrochloric acid. The product was then extracted into ethyl acetate (2 × 50 ml), washed with water (2 × 20 ml), dried (anhydrous $Na_2SO_4$) and evaporated down to leave a dark red oil.

The crude product (400 mg) was dissolved in dry quinoline (2 ml), dry copper bronze added (20 mg) and this mixture was heated under nitrogen at about 200° for 1½ hours. On cooling, the reaction mixture was diluted with ethyl acetate (30 ml) and filtered. The filtrate was washed with dilute aqueous hydrochloric acid (2 × 25 ml) and water (2 × 25 ml), before being dried (anhydrous $Na_2SO_4$) and concentrated to leave a dark red oil.

The crude product was chromatographed on silica (30 g) using 20% ether/petrol as eluant to afford the title compound (D1) as a yellow oil (150 mg, 38%).

NMR: δ ($CDCl_3$) 2.0—2.8 (4H, m), 2.9—3.3 (1H, m), 4.1—4.5 (2H, m). 4.8—5.2 (2H, m), 5.4—6.2 (1H, m), 5.70 (1H, d, J=4Hz), 6.55 (1H, d, J=4Hz), 7.1—7.4 (3H, m), 7.5—7.8 (2H, m).

Description 2

As an alternative to the use of copper/quinoline in the decarboxylation step, the intermediate acid may be heated neat under nitrogen. Accordingly a sample of crude 5-benzoyl-2,3-dihydro-1-(2-propenyl)-1H-pyrrolizine-1-carboxylic acid (35.3 g) prepared by the method given in Description 1, was heated under nitrogen at 200—220° for 1 hour. The required ketone D1 (22.51 g, 75%) was obtained after purification by column chromatography using silica gel (550 g) with pentane/ether as eluant (2:1 rising to 1:1 pentane:ether).

Description 3

[2,3-Dihydro-1-(2-propenyl)-1H-pyrrolizine-5-yl]-(4-chlorophenyl)-methanone

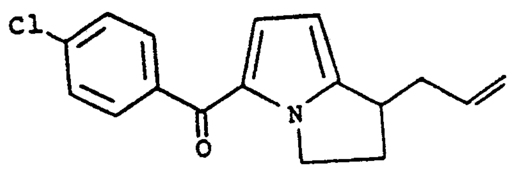

(D3)

This compound was prepared in an analogous manner to the preparation of the compound of Description 1.

NMR: δ ($CDCl_3$) 2.0—2.8 (4H, m); 2.9—3.3 (1H, m); 4.1—4.5 (2H, m); 4.8—5.2 (2H, m); 5.4—6.2 (1H, m); 5.83 (1H, d, J=4Hz); 6.67 (1H, d, J=4Hz); 7.30 (2H, d, J=9Hz); 7.67 (2H, d, J=9Hz).

Example 1

1-(5-benzoyl-2-3-dhydro-1H-pyrrolizin-1-yl)-propan-2-one, (EI)

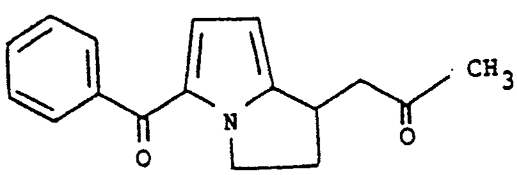

(E1)

Copper (I) chloride (120 mg) and palladium (II) chloride (40 mg) were added to water (0.2 ml) in

dimethylformamide (2 ml) to give a black suspension. Air was bubbled through this mixture for 1 hour with stirring before adding the above propene (D1) (130 mg, 0.00052 mole) in dimethylformamide (2 ml). The mixture was stirred while bubbling air through for 3½ hours.

The reaction mixture was worked up by pouring into water (100 ml) and extracting with ethyl acetate (3 × 30 ml). The combined extracts were washed with water (3 × 20 ml), dried (anhydrous $Na_2SO_4$) and evaporated to dryness to give a red oil.

Column chromatography using silica gel (20 g) and 15% ether/petrol as eluant gave a pale yellow oil, which was crystalliszed from 20% ether/pentane to give the title compound (El) as a white solid (75 mg, 54%), mp 81—83°C.

NMR: δ ($CDCl_3$) 2.12 (3H, s), 2.3—3.1 (4H, m), 3.2—3.7 (1H, m), 4.0—4.5 (2H, m), 5.75 (1H, d, J=4Hz), 6.63 (1H, d, J=4Hz), 7.1—7.4 (3H, m), 7.5—7.8 (2H m).

Observed mass, 267.1254. Calculated mass for $C_{17}H_{17}NO_2$ is 267.1259.

Example 2

1-[5-(4-Chlorobenzoyl)-2,3-dihydro-1H-pyrrolizin-1-yl]propan-2-one, (E2)

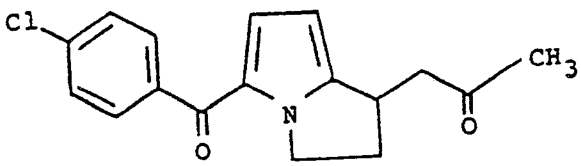

(E2)

Copper (I) chloride (1.66 g, 0.017 mole) and palladium (II) chloride (0.55 g, 0.003 mole) were added to water (3 ml) in dimethylformamide (30 ml) to give a black suspension. Air was bubbled through this mixture for 1 hour with stirring before adding the above propene (D3) (1.8 g, 0.006 mole). The mixture was stirred while bubbling air through for 3½ hours.

The reaction was worked up by pouring into water (300 ml) and extracting with ethyl acetate (3 × 100 ml), dried ($Na_2SO_4$) and evaporated to dryness to give a reddish-brown oil.

Purification by column chromatography on silica gel eluting with 1:1 petroleum ether 60—80°: ether afforded the title compound as beige solid (0.80 g) m.p. 70—72°C (after recrystallisation from ether/pentane).

NMR: δ ($CDCl_3$) 2.15 (3H, s), 2.5—3.1 (4H, m), 3.35—3.85 (1H, m); 4.1—4.6 (2H, m), 5.83 (1H, d, J=4Hz); 6.67 (1H, d, J=4Hz); 7.30 (2H, d, J=9Hz); 7.67 (2H, d, J=9Hz).

Example 3

1-(5-Benzoyl-2,3-dihydro-1H-pyrrolizin-1-yl)propan-2-ol, (E3)

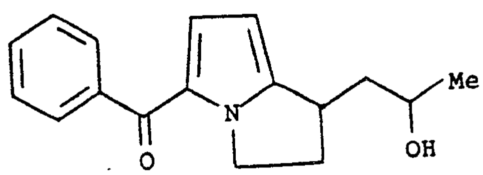

(E3)

The ketone E1 (1 g, 0.0037 mole) in ethanol (200 ml) was cooled to −25° and treated with sodium borohyride (160 mg, 0.0042 mole). The mixture was stirred for 7 mins at this temperature and then quenched by addition of excess aqueous ammonium chloride solution (100 ml). The ethanol was removed under reduced pressure and the remaining aqueous emulsion extracted with ethyl acetate (3 × 100 ml), the combined organic layers washed with water (2 × 100 ml) before drying with anhydrous sodium sulphate. After filtration and removal of solvents under reduced pressure the product was obtained as a pale yellow oil (0.96 g). Chromatography of this material on silica gel (50 g) with ether as eluant gave the required alcohol E3 as a colourless oil (0.84 g, 83%). From the nmr spectrum the product consists of a mixture of two diastereoisomers.

NMR: δ ($CDCl_3$) 1.1—1.4 (3H, two overlapping d, J=6Hz), 1.4—4.8 (8H, multiplets), 2.37 (1H, s, exchanges with $D_2O$), 5.86 (1H, overlapping doublets, J=4Hz), 6.71 (1H, d, J=4Hz), 7.2—7.5 (3H, m), 7.5—7.75 (2H, m).

Example 4

2-Acetoxy-1-(5-benzoyl-2,3-dihydro-1H-pyrrolizin-1-yl)propane, (E4)

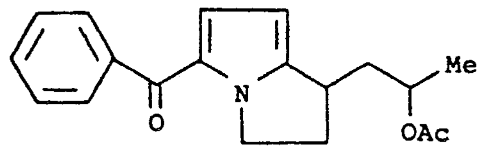

(E4)

The alcohol E3 (0.74 g, 0.00275 mole) was dissolved in dry toluene (25 ml) with added pyridine (1.25 ml). This solution was cooled to 0° in an ice-bath and then acetyl chloride (0.63 ml, 0.0084 mole) was added dropwise. The resulting suspension was stirred at room temperture for 2 hours and then poured into water (50 ml) and the aqueous layer separated and extracted with ether (2 × 50 ml). The combined organic layers were washed with water (30 ml), 5N hydrochloric acid (30 ml) and finally water (2 × 30 ml). After drying with anhydrous sodium sulphate, filtration, and removal of solvents under reduced pressure a yellow oil was obtained. Purification by column chromatography using silica gel (40 g) with ether as eluant gave the required acetate E4 as a pale yellow oil (0.847, 99%).

NMR: δ ($CDCl_3$) 1.2—1.4 (3H, two overlapping d), 1.5—3.5 (5H, multiplets), 2.08 (3H, s), 4—4.8 (2H, m), 4.9—5.4 (1H, m), 5.98 (1H, overlapping doublets, J=4Hz), 6.8 (1H, d, J=4Hz), 7.35—7.6 (3H, m), 7.7—8.0 (2H, m).

This sample is a ~ 1:1 mixture of diastereoisomers.

Example 5

1-(5-Benoyl-2,3-dihydro-1H-pyrrolizin-1-yl)-1-hydroxypropan-2-one, (E5)

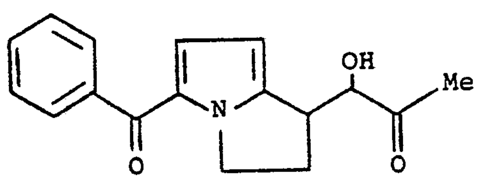

(E5)

The ketone (E1) (1.84 g, 0.00691 mole) was dissolved in methylene dichloride (100 ml) and cooled to −20° under nitrogen. Hexamethyldisilazane (1.34 g, 0.0083 mole) was added with stirring followed by trimethylsilyliodide (1.08 ml, 1.52 g, 0.0076 mole). This mixture was stirred at −20° for 15 minutes and then allowed to warm to room temperature over ½ hour. The reaction was stirred for a further 3½ hours at room temperature when tlc (alumina with ether as eluant) showed almost complete conversion of the propanone to its higher $R_x$ silyl enol ether. The reaction mixture was worked up by rapid treatment with cold saturated aqueous sodium bicarbonate, separation, and drying the organic layer with anhydrous sodium sulphate. After filtration and removal of solvent under reduced pressure the crude silyl enol ether was obtained as an orange oil (2 g).

The crude enol ether was redissolved in methylene dichloride and treated with 85% m-chloroperbenzoic acid (1.54 g, 1.1 equiv.) over a period of about 5 minutes. The mixture was stirred at room temperature for ¾ hour when tlc (alumina with ether as eluant) showed total conversion of the enol ether. The reaction was worked up by addition of aqueous sodium sulphite, separation, washing the organic layer with water, aqueous sodium bicarbonate, water, dilute hydrochloric acid and finally water. Tlc showed only a minor amount of the silyl ether intermediate had been cleaved so THF (150 ml) was added and the acid wash repeated. The resulting mixture after drying with anhydrous sodium sulphate and removal of solvents under reduced pressure gave an orange/brown oil which on tlc (silica gel with ether as eluant) contained some of the starting ketone (E1) and two closely running lower $R_F$ components.

Column chromatography on silica gel (100 g) with ether/pentane as eluant (3:1 pentane:ether, slowly rising to 100% ether) produced a separation of these two components: the higher $R_F$ as a pale yellow oil (369 mg) which solidified on standing, and the lower as a pale yellow oil (345 mg) which more slowly solidifed. Thus the erythro and threo isomers of E5 have been prepared and separated. Higher $R_F$: mp 132—139° without further purification; 140—142° after recrystallisation from chloroform/pentane.

NMR: δ ($CDCl_3$) 2.15—2.65 (2H, m), 2.34 (3H, s), 3.44 (1H, d, J=4Hz, exchanges with $D_2O$), 3.5—3.9 (1H, m), 4.25—4.75 (3H, m), 6.05 (1H, d, J=4Hz), 6.87 (1H, d, J=4Hz), 7.35—7.65 (3H, m), 7.7—8.0 (2H, m).

Lower $R_F$: mp 85—95° without further purification; 97—98° after recrystallisation from chloroform/pentane.

NMR: δ ($CDCl_3$) 2.3 (3H, s), 2.5—3.0 (2H, m), 3.35 (1H, d, J=7Hz, exchanges with $D_2O$), 3.5—3.9 (1H, m), 4.3—4.7 (3H, m), 5.74 (1H, d, J=4Hz), 6.79 (1H, d, J=4Hz), 7.35—7.65 (3H, m), 7.7—7.95 (2H, m).

Example 6

1-(5-Benzoyl-2,3-dihydro-1H-pyrrolizin-1-yl)-1-hydroxypropan-2-ol, (E6)

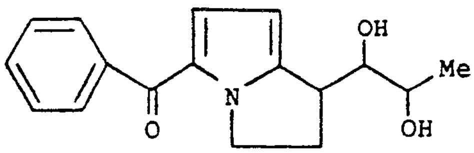

(E6)

A sample of the higher $R_F$ ketol (E5) (118 mg, 0.000417 mole) was dissolved in ethanol (25 ml) and cooled to −25° before adding sodium borohydride (20 mg, 0.00053 mole). The mixture was stirred at this temperature for 7 minutes and then quenched by addition of excess aqueous ammonium chloride solution. The ethanol was removed under reduced pressure and the residual aqueous emulsion extracted with chloroform (3 × 50 ml). The combined organic layers were washed with water (50 ml), dried with anhydrous sodium sulphate and the solvent removed under reduced pressure to give the diol (E6) as a pale yellow oil (118 mg, 99%) which solidified on standing, mp 130—138°.

NMR: δ ($CDCl_3$) 1.1—1.4 (3H, two overlapping d, J=6Hz), 2.15—4.65 (7H, overlapping m), 2.75 (2H, br.s, exchanges with $D_2O$), 5.83 (1H, overlapping doublets, J=4Hz), 6.67 (1H, d, J=4Hz), 7.15—7.5 (3H, m), 7.5—7.85 (2H, m).

This sample is a mixture of diastereoisomers.

Example 7

[2,3-dihydro-1-[(2-methyl-1,3-dioxolan-2-yl)methyl]-1H-pyrrolizin-5-yl]phenyl-methanone

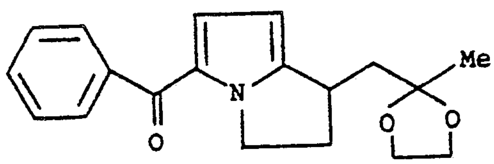

(E7)

A solution of the ketone (E1) (1 g, 0.000375 mole) in toluene (120 ml) with added 1,2-ethanediol (0.61 ml) and p-toluenesulphonic acid (30 mg) was heated overnight at reflux under nitrogen in a Dean and Stark apparatus. Although conversion to the required ketal (E7) was not complete the mixture was evaporated to dryness and the residue chromatographed on silica gel (30 g) with 50% ether/pentane as eluant. The required product was obtained as a pale yellow oil (237 mg, 20%).

NMR: δ ($CDCl_3$) 1.36 (3H, s), 1.7—3.45 (5H, m), 3.9 (4H, s), 3.9—4.7 (2H, m), 5.87 (1H, d, J=4Hz), 6.68 (1H, d, J=4Hz), 7.2—7.5 (3H, m), 7.5—7.8 (2H, m).

1. Activity in the Carrageenin Test

Groups of 8 OLAC Wister male rats (140—170 g) received the compound of Example 1 orally 1 hour before 0.1 ml of 0.5% λ carrageenin injected into the right hind paw. Paw volumes were measured by mercury displacement 3 hours after carrageenin. The results are shown in Table 1.

TABLE 1

| Compound | Dose mg/kg | Paw volume ± s.e. | % Inhibition | $ED_{25}$ mg/kg |
|---|---|---|---|---|
| Methyl cellulose | | 85.8 ± 9.4 | — | |
| Example 1 | 2.5 | ** 45.1 ± 5.4 | 47.4 | |
| Example 1 | 0.5 | 73.4 ± 7.8 | 14.4 | 0.75 |
| Example 1 | 0.1 | 92.6 ± 4.6 | −8.0 | |

** $p < 0.01$ Students 't' test

Significantly different from the control level

2. Gastric Irritancy Test

Groups of OLAC Wistar female rats (130—160 g) were fasted overnight (18 hurs) before receiving orally the compound of Example 1. 4 hours later the stomachs were removed and inspected for the presence of erosion of the gastric muscosa. The results are shown in Table 2.

TABLE 2

| Compound | Dose mg/kg | Proportion of rats with gastric erosions | $ED_{40}$ mg/kg (95% confidence limits |
|---|---|---|---|
| Methyl cellulose | — | 0/4 | — |
| Example 1 | 33.6 | 4/8 | |
| Example 1 | 11.2 | 1/8 | 30.8 (15.4—61.6) |
| Example 1 | 3.7 | 0/8 | |

From these two tests the therapeutic ratio for the compound of formula (E1) was 41.0.

Toxicity

No toxic effects were observed.

**Claims**

1. A compound of the formula (I), or a pharmaceutically acceptable salt thereof,

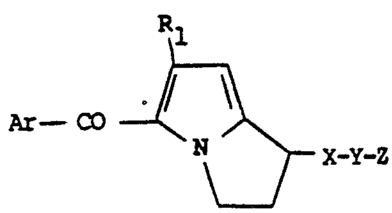

(I)

wherein:

Ar is phenyl optionally substituted in the *o*-, *m*- or *p*-position by $C_{1-4}$ alkyl, $C_{1-4}$ alkoxy, halo, $C_{1-4}$ alkylthio or trifluoromethyl, 2-pyrryl optionally N-substituted by $C_{1-4}$ alkyl, 2-furyl or 2-thienyl either being optionally substituted in the 3-, 4, or 5- position by $C_{1-4}$ alkyl, chloro or bromo, 3-furyl, or 3-thienyl;

$R_1$ is hydrogen, $C_{1-4}$ alkyl, fluoro, bromo or chloro; and

X is $CH_2$ and either Y is CO, CHOH, $CH(OCOR_2)$ or $C(OR_3)OR_4$, and Z is $CH_3$, or Y and Z together are $C(OR_5){=}CH_2$ or $C(OCOR_5){=}CH_2$, or X is CHOH, Y is CHOH or CO and Z is $CH_3$, or X and Y together are $CH{=}C(OR_5)$ or $CH{=}C(OCOR_5)$ and Z is $CH_3$, in which $R_2$ is phenyl optionally substituted in the *o*-, *m*- or *p*-position by $C_{1-4}$ alkyl, $C_{1-4}$ alkoxy or halo, or is $C_{1-7}$ alkyl optionally substituted by phenyl or amino, $R_3$ and $R_4$ are $C_{1-4}$ alkyl or together are $C_{2-3}$ polymethylene, and $R_5$ is $C_{1-4}$ alkyl.

2. A compound according to claim 1, wherein Ar is phenyl optionally substituted by chloro, bromo, methyl, methoxy, trifluoromethyl and methylthio.

3. A compound according to claim 2, wherein Ar is phenyl optionally monosubstituted by methyl or chloro.

4. A compound according to claim 3, wherein Ar is phenyl.

5. A compound according to any one of the preceding claims, wherein X—Y—Z is $CH_2COCH_3$, $CH_2CHOHCH_3$, $CH_2CH(OCOR_2)CH_3$, $CH_2C(OR_3)(OR_4)CH_3$, $CHOHCHOHCH_3$, $CHOHCOCH_3$, in which $R_2$ to $R_4$ are as defined in claim 1.

6. A compound according to claim 5, wherein $R_2$ is methyl, and $R_3$ and $R_4$ together are ethylene.

7. A compound according to any one of the preceding claims, wherein $R_1$ is hydrogen.

8. 1-(5-Benzoyl-2,3-dihydro-1H-pyrrolizin-1-yl)-propan-2-one.

9. 1-[5-(4-Chlorobenzoyl)-2,3-dihydro-1H-pyrrolizin-1-yl]-propan-2-one.

10. 1-(5-Benzoyl-2,3-dihydro-1H-pyrrolizin-1-yl)-propan-2-ol.

11. 2-Acetoxy-1-(5-benzoyl-2,3-dihydro-1H-pyrrolizin-1-yl)-propane.

12. 1-(5-Benzoyl-2,3-dihydro-1H-pyrrolizin-1-yl)-1-hydroxy-propan-2-one.

13. 1-(5-Benzoyl-2,3-dihydro-1H-pyrrolizin-1-yl)-1-hydroxy-propan-2-ol.

14. [2,3-dihydro-1-[(2-methyl-1,3-dioxolan-2-yl)-methyl]-1H-pyrrolizin-5-yl]-phenyl-methanone.

15. A process of preparing a compound of formula (I), as defined in claim 1, which comprises oxidising a compound of formula (II),

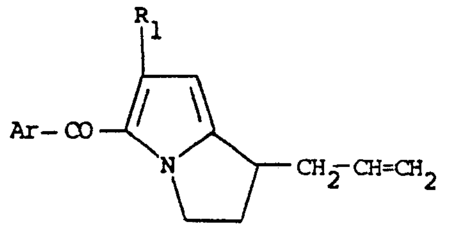

(II)

wherein Ar and $R_1$ are as defined in claim 1, to give a compound of formula (I), wherein X—Y—Z is $CH_2COCH_3$; optionally reacting the resulting compound with:

(a) a reducing agent to give a compound of formula (I), wherein X—Y—Z is $CH_2CHOHCH_3$, and optionally reacting a compound of formula (I); wherein X—Y—Z is $CH_2CHOHCH_3$, with an acylating agent containing the acyl group, $R_2CO$, $R_2$ being as defined in claim 1, to give a compound of formula (I), wherein X—Y—Z is $CH_2CH(OCOR_2)CH_3$;

(b) a ketalising agent containing the alkoxy groups, $OR_3$ and $OR_4$, $R_3$ and $R_4$ being as defined in claim 1, to give a compound of formula (I), wherein X—Y—Z is $CH_2C(OR_3)(OR_4)CH_3$;

(c) an enol $C_{1-4}$ etherifying agent to give compounds of formula (I), wherein X—Y—Z is $CH_2C(OR_5)=CH_2$ and $CH=C(OR_5)CH_3$, $R_5$ being as defined in claim 1, and separating the compound of formula (I), wherein X—Y—Z is $CH_2C(OR_5)CH_2$, from the compound of formula (I), wherein X—Y—Z is $CH=C(OR_5)CH_3$; optionally reacting a compound of formula (I), wherein X—Y—Z is $CH=C(OR_5)CH_3$, with an oxidising agent to give a compound of formula (I), wherein X—Y—Z is $CHOHCOCH_3$, and optionally reacting a compound of formula (I), wherein X—Y—Z is $CHOHCOCH_3$, with a reducing agent to give a compound of formula (I), wherein X—Y—Z is $CHOHCHOHCH_3$;

(d) an enol tri-$C_{1-4}$ alkylsilyl etherifying agent to give a compound of formula (I), wherein X—Y—Z is $CH=C(OR_x)CH_3$, $R_x$ being tri-$C_{1-4}$ alkyl silyl, and reacting the resulting ether with an oxidising agent to give a compound of formula (I), wherein X—Y—Z is $CHOHCOCH_3$; or

(e) an enol $C_{2-5}$ esterifying agent to give compounds of formula (I), wherein X—Y—Z is $CH_2C(OCOR_5)=CH_2$ and $CH=C(OCOR_5)CH_3$, and separating the compound of formula (I) wherein X—Y—Z is $CH_2C(OCOR_5)=CH_2$, from the compound of formula (I) wherein X—Y—Z is $CH=C(OCOR_5)CH_3$, $R_5$ being as defined in claim 1.

16. A process of preparing a compound of formula (I), wherein X—Y—Z is $CH_2C(OR_5)=CH_2$, $R_5$ being as defined in claim 1, which comprises decarboxylating a compound of formula (V),

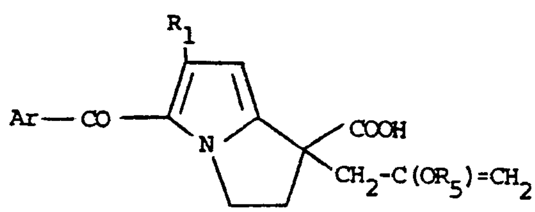

(V)

wherein Ar, $R_1$ and $R_5$ are as defined in claim 1.

17. A pharmaceutical composition, which comprises a compound of formula (I), as defined in any one of claims 1 to 14, and a pharmaceutically acceptable carrier.

18. A compound, as claimed in any one of claims 1 to 14, or a composition, as claimed in claim 17, for use in the treatment of inflammatory and/or painful conditions.

**Patentansprüche**

1. Eine Verbindung der Formel (I) oder deren pharmazeutisch verträgliches Salz

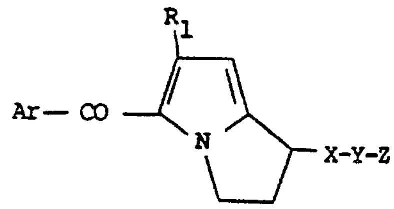

(I)

in der

Ar Phenyl, das gegebenenfalls in *o*-, *m*- oder *p*-Stellung durch $C_{1-4}$-Alkyl, $C_{1-4}$-Alkoxy, Halogen, $C_{1-4}$-Alkylthio oder Trifluormethyl substituiert ist, 2-Pyrryl, das gegebenenfalls durch $C_{1-4}$-Alkyl N-substituiert ist, 2-Furyl oder 2-Thienyl, die gegebenenfalls in der 3-, 4- oder 5-Stellung durch $C_{1-4}$-Alkyl, Chlor oder Brom substituiert sind, 3-Furyl oder 3-Thienyl ist,

$R_1$ Wasserstoff, $C_{1-4}$-Alkyl, Fluor, Brom oder Chlor ist und

X entweder $CH_2$ ist und

Y CO, CHOH, $CH(OCOR_2)$ oder $C(OR_3)OR_4$ ist und

Z $CH_3$ ist oder Y und Z zusammen $C(OR_5)=CH_2$ oder $C(OCOR_5)=CH_2$ sind

oder CHOH ist und

Y CHOH oder CO ist und

Z $H_3$ ist

oder

X und Y zusammen $CH=C(OR_5)$ oder $CH=C(OCOR_5)$ sind und

Z $CH_3$ ist,

wobei $R_2$ Phenyl, das gegebenenfalls in der *o*-, *m*- or *p*-Stellung durch $C_{1-4}$-Alkyl, $C_{1-4}$-Alkoxy oder Halogen substituiert ist, oder $C_{1-7}$-Alkyl ist, das gegebenenfalls durch Phenyl oder Amino substituiert ist,

$R_3$ und $R_4$ $C_{1-4}$-Alkyl oder zusammen $C_{2-3}$-Polymethylen sind und

$R_5$ $C_{1-4}$-Alkyl ist.

2. Eine Verbindung nach Anspruch 1, wobei Ar Phenyl ist, das gegebenenfalls durch Chlor, Brom, Methyl, Methoxy, Trifluormethyl oder Methylthio substituiert ist.

3. Eine Verbindung nach Anspruch 2, wobei Ar Phenyl ist, das gegebenenfalls durch Methyl oder Chlor monosubstituiert ist.

4. Eine Verbindung nach Anspruch 3, wobei Ar Phenyl ist.

5. Eine Verbindung nach irgendeinem der vorstehenden Ansprüche, wobei X—Y—Z $CH_2COCH_3$, $CH_2CHOHCH_3$, $CH_2CH(OCOR_2)CH_3$, $CH_2C(OR_3)(OR_4)CH_3$, $CHOHCHOHCH_3$, $CHOHCOCH_3$ ist, worin $R_2$ bis $R_4$ wie in Anspruch 1 definiert sind.

6. Eine Verbindung nach Anspruch 5, wobei $R_2$ Methyl ist und $R_3$ und $R_4$ zusammen Äthylen sind.

7. Eine Verbindung nach irgendeinem der vorstehenden Ansprüche, wobei $R_1$ Wasserstoff ist.

8. 1-(5-Benzoyl-2,3-dihydro-1H-pyrrolizin-1-yl)-propan-2-on.

9. 1-[5-(4-Chlorbenzoyl)-2,3-dihydro-1H-pyrrolizin-1-yl]-propan-2-on.

10. 1-(5-Benzoyl-2,3-dihydro-1H-pyrrolizin-1-yl)-propan-2-ol.

11. 2-Acetoxy-1-(5-benzoyl-2,3-dihydro-1H-pyrrolizin-1-yl)-propan.

12. 1-(5-Benzoyl-2,3-dihydro-1H-pyrrolizin-1-yl)-1-hydroxy-propan-2-on.

13. 1-(5-Benzoyl-2,3-dihydro-1H-pyrrolizin-1-yl)-1-hydroxy-propan-2-ol.

14. {2,3-Dihydro-1-[(2-methyl-1,3-dioxolan-2-yl)-methyl]-1H-pyrrolizin-5-yl}-phenylmethanon.

15. Ein Verfahren zur Herstellung einer Verbindung der Formel (I), wie in Anspruch 1 definiert, das die Maßnahmen umfaßt:

Oxidieren einer Verbindung der Formel (II)

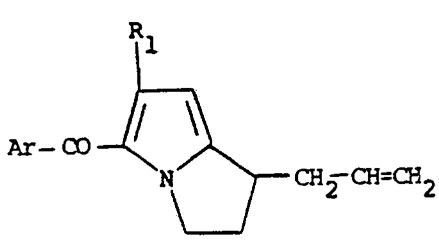

(II)

in der Ar und $R_1$ wie in Anspruch 1 definiert sind, zur Erzeugung einer Verbindung der Formel (I), in der X—Y—Z $CH_2COCH_3$ ist, gegebenenfalls Umsetzen der erhaltenen Verbindung mit

(a) einem reduktionsmittel zur Erzeugung einer Verbindung der Formel (I), in der X—Y—Z $CH_2CHOHCH_3$ ist, und gegebenenfalls Umsetzen einer Verbindung der Formel (I), in der X—Y—Z $CH_2CHOHCH_3$ ist, mit einem Acylierungsmittel, das die Acylgruppe $R_2CO$ enthält, wobei $R_2$ wie in Anspruch 1 definiert ist, zur Erzeugung einer Verbindung, in der X—Y—Z $CH_2CH(OCOR_2)CH_3$ ist;

(b) einem Ketalisierungsmittel, das die Alkoxygruppen $OR_3$ und $OR_4$ enthält, wobei $R_3$ und $R_4$ wie in Anspurch 1 definiert sind, zur Erzeugung einer Verbindung der Formel (I), in der X—Y—Z $CH_2C(OR_3)(OR_4)CH_3$ ist:

(c) einem Enol-1,4-Verätherungsmittel zur Erzeugung von Verbindung der Formel (I), in der X—Y—Z $CH_2C(OR_5)=CH_2$ und $CH=C(OR_5)CH_3$ ist, wobei $R_5$ wie in Anspruch 1 definiert ist, und Abtrennen der Verbindung der Formel (I), in der X—Y—Z $CH_2C(OR_5)CH_2$ ist, von der Verbindung der Formel (I), in der X—Y—Z $CH=C(OR_5)CH_3$ ist, gegebenenfalls Umsetzen einer Verbindung der Formel (I), in der X—Y—Z $CH=C(OR_5)CH_3$ ist, mit einem Oxidationsmittel zur Erzeugung einer Verbindung der Formel (I), in der X—Y—Z $CHOHCOCH_3$ ist, und gegebenenfalls Umsetzen einer Verbindung der Formel (I), in der X—Y—Z $CHOHCOCH_3$ ist, mit einem Reduktionsmittel zur Erzeugung einer Verbindung der Formel (I), in der X—Y—Z $CHOHCHOHCH_3$ ist,

(d) einem Enol-tri-$C_{1-4}$-Alkylsilyl-Verätherungsmittel zur Erzeugung einer Verbindung der Formel (I), in der X—Y—Z $CH=C(OR_x)CH_3$ ist, wobei $R_x$ Tri-$C_{1-4}$-Alkylsilyl ist, und Umsetzen des erhaltenen Äthers mit einem Oxidationsmittel zur Erzeugung einer Verbindung der Formel (I), in der X—Y—Z $CHOHCOCH_3$ ist; oder

(e) einem Enol-$C_{2-5}$-Veresterungsmittel zur Erzeugung von Verbindungen der Formel (I), in der X—Y—Z $CH_2C(OCOR_5)=CH_2$ und $CH=C(OCOR_5)CH_3$ ist, und Abtrennen der Verbindung der Formel (I), in der X—Y—Z $CH_2C(OCOR_5)=CH_2$ ist, von der Verbindung der Formel (I), in der X—Y—Z $CH=C(OCOR_5)CH_3$ ist, wobei $R_5$ wie in Anspruch 1 definiert ist.

16. Ein Verfahren zur Herstellung einer Verbindung der Formel (I), in der X—Y—Z $CH_2C(OR_5)=CH_2$ ist, wobei $R_5$ wie in Anspruch 1 definiert ist, die Maßnahme umfassend einer Decarboxylierung einer Verbindung der Formel (V)

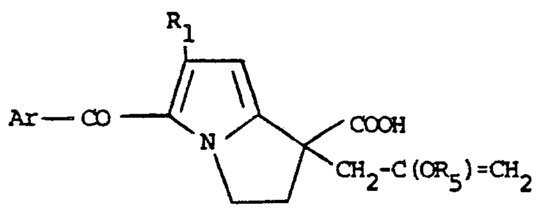

(V)

in der Ar, $R_1$ und $R_5$ wie in Anspruch 1 definiert sind.

17. Eine pharmazeutische Zusammensetzung, umfassend eine Verbindung der Formel (I), wie in irgendeinem der Ansprüche 1 bis 14 definiert, und einen pharmazeutisch verträglichen Träger.

18. Eine Verbindung, wie in irgendeinem der Ansprüche 1 bis 14 definiert, oder eine Zusammensetzung, wie in Anspruch 17 definiert, zur Verwendung bei der Behandlung von Entzündungen und/oder Schmerzen.

## Revendications

1. Composé de formule (I) ou un sel de celui-ci acceptable du point de vue pharmaceutique

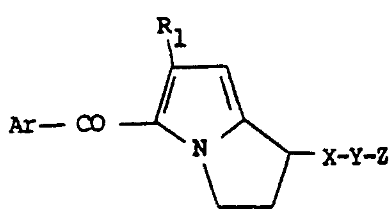

(I)

dans laquelle

Ar est un groupe phényle éventuellement substitué en position *o-*, *m-* or *p-* par un groupe alcoyle en $C_{1-4}$, alcoxy en $C_{1-4}$, halo, alcoylthio en $C_{1-4}$ ou trifluorométhyle, 2-pyrryle éventuellement N-substitué par un groupe alcoyle en $C_{1-4}$, 2-furyle ou 2-thiènyle, l'un ou l'ature éventuellement substitué en position 3-, 4- ou 5- par un groupe alcoyle en $C_{1-4}$, un atome de chlore ou de brome, 3-furyle ou 3-thiènyle; $R_1$ est un atome d'hydrogène, un groupe alcoyle en $C_{1-4}$, un atome de fluor, brome ou chlore; et

X est $CH_3$ et soit Y est CO, CHOH, $CH(OCOR_2)$ ou $C(OR_3)OR_4$, et Z est $CH_3$, soit Y et Z forment ensemble $C(OR_5)=CH_2$ ou $C(OCOR_5)=CH_2$, ou bien X est CHOH, Y est CHOH ou CO et Z est $CH_3$, ou X et Y forment ensemble $CH=C(OR_5)$ ou $CH=C(OCOR_5)$ et Z est $CH_3$, où $R_2$ est un groupe phényle éventuellement

substitué en position *o-*, *m-* or *p-* par un groupe alcoyle en $C_{1-4}$, alcoxy en $C_{1-4}$ u halo, ou il est un groupe alcoyle en $C_{1-7}$ éventuellement substitué par un groupe phényle ou amino, $R_3$ et $R_4$ sont un groupe alcoyle en $C_{1-4}$ ou ils forment ensemble un radical polyméthylène en $C_{2-3}$, et $R_5$ est un groupe alcoyle en $C_{1-4}$.

2. Composé suivant la revendication 1, caractérisé en ce que Ar est un groupe phényle éventuellement substitué par un radical chloro, bromo, méthyle, méthoxy, trifluorométhyle et méthylthio.

3. Composé suivant la revendication 2, caractérisé en ce que Ar est un groupe phényle éventuellement monosubstitué par un radical méthyle ou chloro.

4. Composé suivant la revendication 3, caractérisé en ce que Ar est un groupe phényle.

5. Composé suivant l'une quelconque des revendications précédentes, caractérisé en ce que X—Y—Z est $CH_2COCH_3$, $CH_2CHOHCH_3$, $CH_2CH(OCOR_2)CH_3$, $CH_2C(OR_3)(OR_4)CH_3$, $CHOHCHOHCH_3$, $CHOHCOCH_3$, où $R_2$ à $R_4$ sont tels que définis dans la revendication 1.

6. Composé suivant la revendication 5, caractérisé en ce que $R_2$ est un groupe méthyle et $R_3$ et $R_4$ forment ensemble un radical éthylène.

7. Composé suivant l'une quelconque des revendications précédentes, caractérisé en ce que $R_1$ est un atome d'hydrogène.

8. 1-(5-Benzoyl-2,3-dihydro-1H-pyrrolizin-1-yl)-propan-2-one.

9. 1-[5-(4-Chlorobenzoyl)-2,3-dihydro-1H-pyrrolizin-1-yl]-propan-2-one.

10. 1-(5-Benzoyl-2,3-dihydro-1H-pyrrolizin-1-yl)-propan-2-ol.

11. 2-Acétoxy-1-(5-benzoyl-2,3-dihydro-1H-pyrrolizin-1-yl)-propane.

12. 1-(5-Benzoyl-2,3-dihydro-1H-pyrrolizin-1-yl)-1-hydroxy-propan-2-one.

13. 1-(5-Benzoyl-2,3-dihydro-1H-pyrrolizin-1-yl)-1-hydroxy-propan-2-ol.

14. [2,3-Dihydro-1-/(2-méthyl-1,3-dioxolan-2-yl)-méthyl]-1H-pyrrolizin-5-yl/-phényl-méthanone.

15. Procédé de préparation d'un composé de formule (I), tel que défini dans la revendication 1, caractérisé en ce que l'on oxyde un composé de formule (II)

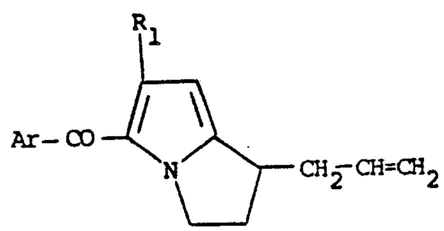

(II)

dans laquelle Ar et $R_1$ sont tels que définis dans la revendication 1, pour obtenir un composé de formule (I), où X—Y—Z est un groupe $CH_2COCH_3$; on fait éventuellement réagir le composé résultant avec:

(a) un agent réducteur pour donner un composé de formule (I) où X—Y—Z est $CH_2CHOHCH_3$ et l'on fait réagir éventuellement un composé de formule (I) où X—Y—Z est $CH_2CHOHCH_3$ avec un agent acylant contenant le groupe acyle, $R_2CO$, $R_2$ étant tel que défini dans la revendication 1, pour donner un composé de formule (I), où X—Y—Z est $CH_2CH(OCOR_2)CH_3$;

(b) un agent cétalisant contenant les groupes alcoxy $OR_3$ et $OR_4$, $R_3$ et $R_4$ étant tels que définis dans la revendication 1, pour donner un composé de formule (I) où X—Y—Z est $CH_2C(OR_3)(OR_4)CH_3$;

(c) un agent éthérifiant ($C_{1-4}$) l'énol pour donner des composés de formule (I) où X—Y—Z est $CH_2C(OR_5)=CH_2$ et $CH=C(OR_5)CH_3$, $R_5$ étant tel que défini dans la revendication 1, et l'on sépare le composé de formule (I) où X—Y—Z est $CH_2C(OR_5)=CH_2$ du composé de formule (I), où X—Y—Z est $CH_2CH=C(OR_5)CH_3$; on fait éventuellement réagir un composé de formule (I), où X—Y—Z est $CH=C(OR_5)CH_3$, avec un agent oxydant pour donner un composé de formule (I) où X—Y—Z est $CHOHCOCH_3$ et l'on fait éventuellement réagir un composé de formule (I) où X—Y—Z est $CHOHCHOHCH_3$;

(d) un agent éthérifiant tri-alcoyl($C_{1-4}$)silylique réagissant sur l'énol pour donner un composé de formule (I) où X—Y—Z est $CH=C(OR_x)CH_3$, $R_x$ étant un groupe tri-alcoyl($C_{1-4}$)silyle et l'on fait réagir l'éther résultant avec un agent oxydant pour donner un composé de formule (I) où X—Y—Z est $CHOHCOCH_3$; ou

(e) un agent estérifiant ($C_{2-5}$) l'énol pour donner des composé de formule (I) où X—Y—Z est $CH_2C(OCOR_5)=CH_2$ et $CH=C(OCOR_5)CH_3$, et l'on sépare le composé de formule (I) où X—Y—Z est $CH_2C(OCOR_5)=CH_2$ du composé de formula (I) où X—Y—Z est $CH=C(OCOR_5)CH_3$, $R_5$ étant tel que défini dans la revendication 1.

16. Procédé de préparation d'un composé de formule (I) où X—Y—Z est $CH_2C(OR_5)=CH_2$, $R_5$ étant tel que défini dans la revendication 1, caractérisé en ce qu'on décarboxyle un composé de formule (V)

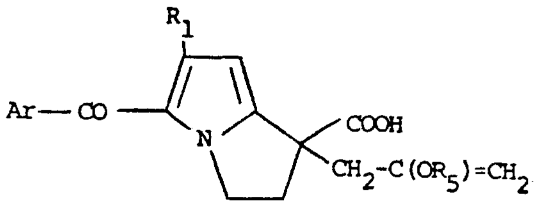

(V)

dans laquelle AR, $R_1$ et $R_5$ sont tels que définis dans la revendication 1.

17. Composition pharmaceutique, caractérisée en ce qu'elle comprend un composé de formule (I), tel qu'il est défini dans l'une quelconque des revendicatins 1 à 14, et un support acceptable du point de vue pharmaceutique.

18. Composé suivant l'une quelconque des revendications 1 à 14 ou une composition suivant la revendication 17, utile dans le traitement d'états inflammatoires et/ou douloureux.